(19)

Europäisches
Patentamt
European
Patent Office
Office européen
des brevets

(11) **EP 4 245 149 A1**

(12) **EUROPEAN PATENT APPLICATION**
published in accordance with Art. 153(4) EPC

(43) Date of publication:
**20.09.2023 Bulletin 2023/38**

(21) Application number: **21891968.6**

(22) Date of filing: **11.11.2021**

(51) International Patent Classification (IPC):
*A23J 3/14* (2006.01)      *A23J 3/34* (2006.01)
*A23L 25/00* (2016.01)     *A23L 11/60* (2021.01)
*A23L 2/66* (2006.01)      *A23L 2/38* (2021.01)
*A23L 7/10* (2016.01)      *A23L 29/00* (2016.01)

(52) Cooperative Patent Classification (CPC):
**A23J 3/14; A23J 3/34; A23L 2/38; A23L 2/66;**
**A23L 7/10; A23L 11/60; A23L 25/00; A23L 29/00**

(86) International application number:
**PCT/JP2021/041607**

(87) International publication number:
**WO 2022/102723 (19.05.2022 Gazette 2022/20)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **11.11.2020  JP 2020188276**
**11.12.2020  JP 2020205795**
**12.03.2021  JP 2021040652**

(71) Applicants:
• **Amano Enzyme Inc.**
**Nagoya-shi**
**Aichi 460-8630 (JP)**

• **Amano Enzyme Europe Ltd.**
**Oxfordshire, OX75SR (GB)**

(72) Inventors:
• **TAKAHASHI, Akiko**
**Kakamigahara-shi, Gifu 509-0109 (JP)**
• **FUJIOKA, Hiroki**
**Chipping Norton, Oxfordshire, OX75SR (GB)**
• **HIURA, Keita**
**Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

(54) **METHOD FOR PRODUCING PLANT-DERIVED PROCESSED PROTEIN-CONTAINING LIQUID COMPOSITION**

(57)    The purpose of the present invention is to provide a processing technology that exhibits an excellent solubilization effect on a plant-based protein-containing liquid composition. This method for producing a processed plant-based protein-containing liquid composition comprises a step for processing a plant-based protein-containing liquid composition by using a protein deamidase and a protease. The processed plant-based protein-containing liquid composition obtained from said production method has increased solubility.

EP 4 245 149 A1

**Description**

TECHNICAL FIELD

The present invention relates to a method for producing a processed plant protein-containing liquid composition, and more specifically to a method for producing a plant protein-containing liquid composition processed to have improved solubility.

BACKGROUND ART

Plant protein beverages, which are rich in nutrients and can be stored for a long period of time, have been increasingly popular as substitutes for animal milks for various reasons such as recent health boom, countermeasures against allergic problems, religious reasons, and increased voluntary restraint of going outdoors accompanying the spread of infectious diseases and the like.

Meanwhile, plant proteins generally have lower solubility and the like than proteins contained in animal milks, and thus use of plant proteins is inevitably limited. Therefore, plant milks cannot sufficiently substitute for animal milks, and use or application of plant milks is not sufficiently achieved.

A method known as effective for improving the solubility of a plant protein is deamidation, that is, hydrolysis of an amide group in a side chain of a glutamine residue or an asparagine residue in a protein. For example, Patent Document 1 discloses that aggregation of a plant milk added to a high-temperature liquid food or beverage can be suppressed by treating the plant milk with a protein deamidase.

PRIOR ART DOCUMENT

PATENT DOCUMENT

Patent Document 1: WO 2020/171106 A

SUMMARY OF THE INVENTION

PROBLEMS TO BE SOLVED BY THE INVENTION

In order to allow plant protein beverages (plant protein-containing liquid compositions) to penetrate more widely and deeply into the general market, further improvement of processing techniques is awaited. The present inventors have focused on solubility as a property of a plant protein-containing liquid composition to be controlled by such processing techniques. However, at present, a processing technique is unknown that achieves a satisfactory solubilization effect on a plant protein-containing liquid composition.

Therefore, an object of the present invention is to provide a processing technique that achieves an excellent solubilization effect on a plant protein-containing liquid composition.

MEANS FOR SOLVING THE PROBLEM

The present inventors have found that the solubility of a plant protein-containing liquid composition is dramatically improved by treating the composition with a protease and a protein deamidase. Furthermore, the present inventors have unexpectedly found that when a specific protease is used, a change in taste can be suppressed while the solubility is improved. The present invention has been completed by further studies based on the above-described findings.

That is, the present invention provides the invention of the aspects described below.

Item 1. A method for producing a processed plant protein-containing liquid composition, the method including a step of treating a plant protein-containing liquid composition with a protease and a protein deamidase.

Item 2. The method according to the item 1, wherein the plant protein-containing liquid composition is treated with the protease and then treated with the protein deamidase.

Item 3. The method according to the item 1 or 2, wherein the protease is a protease derived from a filamentous fungus.

Item 4. The method according to any one of the items 1 to 3, wherein the protease is derived from Aspergillus oryzae.

Item 5. The method according to any one of the items 1 to 4, wherein the plant protein contains a protein of a plant selected from the group consisting of oats, peas, chickpeas, rice, and almonds.

Item 6. The method according to the items 1 to 5, wherein the plant protein-containing liquid composition is a plant milk.

Item 7. A solubilizer for a plant protein-containing liquid composition, the solubilizer including a protease and a protein deamidase.

Item 8. A solubilizer including a neutral protease, the solubilizer to be used for solubilizing a plant protein-containing liquid composition to be treated with a protein deamidase while a change in taste of the plant protein-containing liquid composition is suppressed.

Item 9. A solubilizer including a protease derived from a filamentous fungus, the solubilizer to be used for solubilizing a plant protein-containing liquid composition to be treated with a protein deamidase while a change in taste of the plant protein-containing liquid composition is suppressed.

ADVANTAGES OF THE INVENTION

According to the present invention, a processing technique is provided that achieves an excellent solubilization effect on a plant protein-containing liquid composition.

EMBODIMENTS OF THE INVENTION

1. Method for Producing Processed Plant Protein-Containing Liquid Composition

The method for producing a processed plant protein-containing liquid composition of the present invention includes a step of treating a plant protein-containing liquid composition with a protease and a protein deamidase. Hereinafter, the method for producing a processed plant protein-containing liquid composition of the present invention will be described in detail.

1-1. Plant Protein-Containing Liquid Composition

The plant protein-containing liquid composition used in the present invention is not particularly limited as long as it is a liquid in which a plant protein is dissolved and/or dispersed in water. Specific examples of the plant protein-containing liquid composition include (i) liquids obtained by dispersing a dry powder of an ingredient containing a plant protein (preferably a plant food ingredient) in water, (ii) liquids obtained by crushing and dispersing an ingredient containing a plant protein (preferably a plant food ingredient) in water and, as necessary, removing an insoluble matter derived from a food ingredient skin or the like by any means such as centrifugal filtration, filtration, a filter bag, or a sieve, (iii) liquids in which the plant protein content is increased by, for example, removing a component other than the plant protein from a liquid of (i) or (ii) described above, and (iv) liquids obtained by dissolving and/or dispersing a dry powder prepared from any liquid of (i) to (iii) in water.

The plant protein is not particularly limited, and examples of the plant protein include proteins of plants (plant food ingredients) such as cereals such as oats, barley, wheat, rice, buckwheat, barnyard millet, millet, teff, and quinoa, pulses such as soybeans, peas, lupins, broad beans, and chickpeas, and nuts such as canary seeds, linseed, almonds, cashew nuts, hazelnuts, pecan nuts, macadamia nuts, pistachios, walnuts, Brazil nuts, peanuts, coconuts, chestnuts, sesame, and pine nuts. These plant proteins may be used singly or in combination of two or more thereof.

Among these plant proteins, proteins of oats, peas, chickpeas, rice, and almonds are preferable from the viewpoint of further enhancing the effect of improving the solubility. Furthermore, proteins of peas, chickpeas, rice, and almonds are preferable when a protease derived from a filamentous fungus is used as the protease, from the viewpoint of further enhancing the effect of improving the solubility and/or the effect of suppressing a change in taste.

Preferred examples of the plant protein-containing liquid composition include plant milks prepared from plant food ingredients. Preferred examples of the plant milks preferably include oat milks, pea milks, chickpea milks, rice milks, and almond milks from the viewpoint of further enhancing the effect of improving the solubility. Examples of the oat milks include oat milks having a form of heat-treated oat slurry, and the temperature of the heat treatment is, for example, 55 to 100°C, preferably 57 to 80°C, more preferably 59 to 70°C, and still more preferably 59 to 65°C. Furthermore, examples of the plant milks preferably include pea milks, chickpea milks, rice milks, and almond milks when a protease derived from a filamentous fungus is used as the protease, from the viewpoint of further enhancing the effect of improving the solubility and/or the effect of suppressing a change in taste.

The protein content in the plant protein-containing liquid composition used in the present invention is not particularly limited.

The protein content in the plant protein-containing liquid composition is, for example, 0.1 to 8 wt% and preferably 0.5 to 5 wt%. More specifically, the oat protein content in the oat protein-containing liquid composition is preferably 0.5 to 4 wt% and more preferably 1 to 3 wt%, the pea protein content in the pea protein-containing liquid composition is preferably 1 to 5 wt% and more preferably 2 to 4 wt%, the chickpea protein content in the chickpea protein-containing liquid composition is preferably 0.5 to 4 wt% and more preferably 1 to 3 wt%, the rice protein content in the rice protein-containing liquid composition is preferably 0.5 to 4 wt% and more preferably 1 to 3 wt%, and the almond protein content in the almond protein-containing liquid composition is preferably 1 to 5 wt% and more preferably 2 to 4 wt%.

As another example, the protein content in the plant protein-containing liquid composition is such that the amount of water used with respect to 1 part by weight of an ingredient containing a plant protein (preferably a plant food ingredient) is, for example, 2 to 30 parts by weight, preferably 3 to 25 parts by weight, and more preferably 6 to 12 parts by weight. More specifically, the oat protein content in the oat protein-containing liquid composition is such that the amount of water used with respect to 1 part by weight of the oat (in terms of the amount of whole oat grains) is, for example, 6 to 12 parts by weight, preferably 8 to 10 parts by weight, and more preferably 8.5 to 9.5 parts by weight.

As the plant protein-containing liquid composition that contains a cereal protein, a composition treated with α-amylase is preferably used from the viewpoint of improving the solubility of the processed plant protein-containing liquid composition produced by the present invention. The α-amylase is not particularly limited, and is, for example, derived from the genus Aspergillus or the genus Bacillus, and is preferably derived from the genus Bacillus and α-amylase of Bacillus subtilis, Bacillus amyloliquefaciens, or Bacillus licheniformis, and is more preferably α-amylase of Bacillus amyloliquefaciens. The amount of the α-amylase used with respect to 1 part by weight of the cereal (in terms of the amount of whole cereal grains) is, for example, 5 to 300 U. In a case where the cereal is an oat, the amount of the α-amylase used with respect to 1 part by weight of the oat (in terms of the amount of whole oat grains) is, for example, 5 to 300 U, preferably 10 to 150 U, more preferably 20 to 70 U, and still more preferably 30 to 50 U. The activity of α-amylase is defined so that the amount of the enzyme that reduces coloring of potato starch due to iodine by 10% per minute is 1 unit (1U).

1-2. Protein Deamidase

The protein deamidase used in the present invention is an enzyme that exhibits an action of decomposing an amide group-containing side chain of a protein without cleavage of a peptide bond and crosslinking of the protein, and the type, the origin, and the like of the enzyme are not particularly limited. As long as the protein deamidase exhibits the above action as main activity, the protein deamidase may further have an action of decomposing an amide group-containing side chain of a protein with cleavage of a peptide bond and crosslinking of the protein.

Examples of the protein deamidase include enzymes that deamidate a glutamine residue in a protein and convert the residue into a glutamic acid residue (such as protein-glutaminases) and enzymes that deamidate an asparagine residue in a protein and convert the residue into an aspartic acid residue (such as protein-asparaginases).

More specific examples of the protein deamidase include protein deamidases derived from the genera Chryseobacterium, Flavobacterium, Empedobacter, Sphingobacterium, Aureobacterium, Myroides, Luteimicrobium, Agromyces, Microbacterium, and Leifsonia. These protein deamidases are known, and for example, JP 2000-50887 A, JP 2001-218590 A, WO 2006/075772 A1, and WO 2015/133590 can be referred to. These protein deamidases may be used singly or in combination of two or more thereof.

Among these protein deamidases, protein deamidases derived from the genus Chryseobacterium are preferable, protein-glutaminases derived from the genus Chryseobacterium are more preferable, protein-glutaminases derived from Chryseobacterium proteolyticum are still more preferable, and a protein-glutaminase derived from Chryseobacterium proteolyticum strain 9670 is still even more preferable from the viewpoint of further improving the solubilization effect or further improving the solubilization effect and the property of suppressing a change in taste.

The protein deamidase can be prepared from a culture liquid of a microorganism as an origin of the protein deamidase. Specific examples of the preparation method include a method in which the protein deamidase is recovered from a culture liquid or a bacterial cell of the above-described microorganism. For example, in the case of using a microorganism that secretes a protein deamidase, a bacterial cell is previously recovered from a culture liquid by, as necessary, filtration, centrifugation, or the like, and then an enzyme can be separated and/or purified. In the case of using a microorganism that secretes no protein deamidase, a bacterial cell is previously recovered from a culture liquid as necessary and then disrupted by pressure treatment, ultrasonic treatment, or the like to expose an enzyme, and then the enzyme can be separated and/or purified. As the method of separation and/or purification of an enzyme, a known method of separation and/or purification of a protein can be used without particular limitation, and examples of the method include a centrifugal separation method, an ultrafiltration (UF) concentration method, a salting-out method, and various chromatography methods using an ion exchange resin. The separated and/or purified enzyme can be powderized with a drying method such as freeze-drying or reduced-pressure drying, and can also be powderized using an appropriate excipient and/or drying aid in the drying method. The separated and/or purified enzyme can also be liquefied by addition of an appropriate additive and filtration sterilization.

As the protein deamidase, a commercially available product can also be used, and examples of a preferred commercially available product include a protein-glutaminase "Amano" 500 (derived from Chryseobacterium proteolyticum) manufactured by Amano Enzyme Inc.

The amount of the protein deamidase used is not particularly limited, and is, for example, 0.01 U or more with respect to 1 g of the plant protein. The amount of the protein deamidase used with respect to 1 g of the plant protein is preferably 0.05 U or more or 0.1 U or more, more preferably 0.5 U or more, still more preferably 0.8 U or more, even more preferably 1 U or more, and still even more preferably 1.5 U or more, 2 U or more, 2.5 U or more, or 2.8 U or more from the viewpoint of further improving the solubilization effect or further improving the solubilization effect and the property of suppressing a change in taste. The upper limit of the range of the amount of the protein deamidase used with respect to 1 g of the plant protein is not particularly limited, and is, for example, 40 U or less, 30 U or less, 20 U or less, 15 U or less, 10 U or less, 5 U or less, 4 U or less, 3.2 U or less, or 3 U or less.

More specifically, in a case where the plant protein is an oat protein, the preferred amount of the protein deamidase used with respect to 1 g of the oat protein is, for example, 0.1 U or more, 0.5 U or more, or 1 U or more, preferably 1.5 U or more, more preferably 2 U or more, still more preferably 2.5 U or more, and still even more preferably 2.8 U or more from the viewpoint of further improving the solubilization effect or further improving the solubilization effect and the property of suppressing a change in taste. The preferred upper limit of the range of the amount of the protein deamidase used with respect to 1 g of the oat protein is, for example, 40 U or less, 30 U or less, 20 U or less, 10 U or less, 5 U or less, 4 U or less, or 3.2 U or less.

In a case where the plant protein is a pea protein, a chickpea protein, a rice protein, and/or an almond protein, the preferred amount of the protein deamidase used with respect to 1 g of these plant proteins is, for example, 0.1 U or more, 0.5 U or more, or 1 U or more, preferably 1.5 U or more, and more preferably 2 U or more from the viewpoint of further improving the solubilization effect or further improving the solubilization effect and the property of suppressing a change in taste. The preferred upper limit of the range of the amount of the protein deamidase used with respect to 1 g of these plant proteins is, for example, 40 U or less, 30 U or less, 20 U or less, 10 U or less, 5 U or less, 4 U or less, or 3 U or less.

The amount of the protein deamidase used with respect to 1 g of the plant protein ingredient is, for example, 0.001 U or more. The preferred amount of the protein deamidase used with respect to 1 g of the plant protein ingredient is 0.005 U or more or 0.01 U or more, and more preferably 0.05 U or more, 0.1 U or more, 0.15 U or more, 0.3 U or more, 0.35 U or more, 0.4 U or more, 0.5 U or more, 1 U or more, or 1.5 U or more from the viewpoint of further improving the solubilization effect on the plant protein-containing liquid composition or further improving the solubilization effect and the property of suppressing a change in taste. The upper limit of the range of the amount of the protein deamidase used with respect to 1 g of the plant protein ingredient is not particularly limited, and is, for example, 20 U or less, 10 U or less, 5 U or less, 4 U or less, 3 U or less, 2 U or less, 1.5 U or less, 1 U or less, 0.6 U or less, 0.5 U or less, 0.45 U or less, 0.4 U or less, or 0.3 U or less.

More specifically, in a case where the plant protein is an oat protein, the preferred amount of the protein deamidase used with respect to 1 g of the oat (in terms of the amount of whole oat grains) is, for example, 0.05 U or more, preferably 0.1 U or more, more preferably 0.15 U or more, still more preferably 0.3 U or more, and still even more preferably 0.35 U or more from the viewpoint of further improving the solubilization effect or further improving the solubilization effect and the property of suppressing a change in taste. The preferred upper limit of the range of the amount of the protein deamidase used with respect to 1 g of the oat (in terms of the amount of whole oat grains) is, for example, 4 U or less, 3 U or less, 2 U or less, 1 U or less, 0.6 U or less, 0.4 U or less, or 0.45 U or less.

More specifically, in a case where the plant protein is a rice protein, the preferred amount of the protein deamidase used with respect to 1 g of the rice (in terms of the amount of dry unpolished rice powder) is, for example, 0.01 U or more or 0.05 U or more, preferably 0.1 U or more, and more preferably 0.15 U or more from the viewpoint of further improving the solubilization effect or further improving the solubilization effect and the property of suppressing a change in taste. The preferred upper limit of the range of the amount of the protein deamidase used with respect to 1 g of the rice (in terms of the amount of dry unpolished rice powder) is, for example, 2 U or less, 1 U or less, 0.5 U or less, or 0.3 U or less.

In a case where the plant protein is an almond protein, the preferred amount of the protein deamidase used with respect to 1 g of the almond (in terms of the amount of almond powder) is, for example, 0.05 U or more or 0.1 U or more, preferably 0.3 U or more, and more preferably 0.4 U or more from the viewpoint of further improving the solubilization effect or further improving the solubilization effect and the property of suppressing a change in taste. The preferred upper limit of the range of the amount of the protein deamidase used with respect to 1 g of the almond (in terms of the amount of almond powder) is, for example, 5 U or less, 4 U or less, 2 U or less, 1 U or less, or 0.6 U or less.

In a case where the plant protein is a chickpea protein, the preferred amount of the protein deamidase used with respect to 1 g of the chickpea is, for example, 0.05 U or more or 0.1 U or more, preferably 0.3 U or more, and more preferably 0.4 U or more from the viewpoint of further improving the solubilization effect or further improving the solubilization effect and the property of suppressing a change in taste. The preferred upper limit of the range of the amount of the protein deamidase used with respect to 1 g of the chickpea is, for example, 5 U or less, 4 U or less, 2 U or less, 1 U or less, or 0.6 U or less.

In a case where the plant protein is a pea protein, the preferred amount of the protein deamidase used with respect to 1 g of the pea is, for example, 0.2 U or more or 0.5 U or more, preferably 1 U or more, and more preferably 1.5 U or more from the viewpoint of further improving the solubilization effect or further improving the solubilization effect and the property of suppressing a change in taste. The preferred upper limit of the range of the amount of the protein deamidase used with respect to 1 g of the pea is, for example, 20 U or less, 10 U or less, 5 U or less, or 3 U or less.

The activity of the protein deamidase is defined so that when benzyloxycarbonyl-L-glutaminylglycine (Z-Gln-Gly) is used as a substrate, the amount of enzyme that liberates 1 $\mu$mol of ammonia per minute is 1 unit (1 U).

1-3. Protease

The protease used in the present invention is not particularly limited as long as it is an enzyme that hydrolyzes a peptide bond of a protein.

Examples of the protease include a protease derived from a filamentous fungus and a protease derived from a bacterium in accordance with the classification based on origin. One of these proteases may be used, or both of them may be used in combination.

The protease derived from a filamentous fungus is not particularly limited as long as a desired effect of the present invention can be obtained. Specific examples of the protease derived from a filamentous fungus include proteases derived from the genera Aspergillus, Mucor, Neurospora, Penicillium, Rhizomucor, Rhizopus, Sclerotinia, and the like. These proteases derived from a filamentous fungus may be used singly or in combination of two or more thereof.

Specific examples of the protease derived from the genus Aspergillus include proteases derived from Aspergillus oryzae, Aspergillus niger, Aspergillus melleus, Aspergillus japonicus, Aspergillus awamori, Aspergillus kawachii, Aspergillus sojae, Aspergillus tamarii, Aspergillus foetidus, Aspergillus fumigatus, Aspergillus nidulans, Aspergillus aculeatus, Aspergillus candidus, Aspergillus flavus, Aspergillus saitoi, Aspergillus inuii, Aspergillus glaucus, Aspergillus caesiellus, Aspergillus clavatus, Aspergillus deflectus, Aspergillus fischerianus, Aspergillus parasiticus, Aspergillus penicilloides, Aspergillus restrictus, Aspergillus sydowii, Aspergillus terreus, Aspergillus ustus, Aspergillus versicolor, and the like. These proteases derived from the genus Aspergillus may be used singly or in combination of two or more thereof.

Examples of the protease derived from a bacterium include proteases derived from the genera Bacillus (or Geobacillus) and the like. These proteases derived from a bacterium may be used singly or in combination of two or more thereof.

Specific examples of the proteases derived from the genus Bacillus (or Geobacillus) include proteases derived from Bacillus amyloliquefaciens, Bacillus cereus, Bacillus clausii, Bacillus intermedius, Bacillus lentus, Bacillus licheniformis, Bacillus stearothermophilus, Bacillus subtilis, Bacillus thermoproteolyticus, and these species belonging to the genus Geobacillus. Among these proteases derived from the genus Bacillus (or Geobacillus), proteases derived from Bacillus stearothermophilus are preferable, and proteases derived from Geobacillus stearothermophilus are more preferable, from the viewpoint of further improving the solubilization effect on the plant protein-containing liquid composition.

Among the above proteases, proteases derived from a filamentous fungus and proteases derived from a bacterium are preferable from the viewpoint of further enhancing the effect of improving the solubility. Among the above proteases, proteases derived from a filamentous fungus are preferable from the viewpoint of further obtaining an effect of suppressing a change in taste. In a case where the plant protein is an oat protein, either a protease derived from a filamentous fungus or a protease derived from a bacterium is preferably used from the viewpoint of further enhancing the effect of improving the solubility and/or further obtaining an effect of suppressing a change in taste, and in a case where the plant protein is a pea protein, a chickpea protein, a rice protein, and/or an almond protein, a protease derived from a filamentous fungus is preferably used from the viewpoint of further improving the effect of suppressing a change in taste or from the viewpoint of further improving the effect of suppressing a change in taste and further enhancing the effect of improving the solubility.

Among the above proteases derived from a filamentous fungus, proteases derived from the genus Aspergillus are preferable from the viewpoint of further enhancing the effect of improving the solubility and/or the effect of suppressing a change in taste. Furthermore, among the above proteases derived from the genus Aspergillus, proteases derived from Aspergillus oryzae are preferable from the viewpoint of further enhancing the effect of improving the solubility and/or the effect of suppressing a change in taste. In a case where the plant protein is an oat protein, among the above proteases derived from the genus Aspergillus, proteases derived from Aspergillus niger and Aspergillus oryzae are preferable, and proteases derived from Aspergillus oryzae are more preferable, from the viewpoint of further improving the solubilization effect on the plant protein-containing liquid composition.

Examples of the protease include acidic proteases, neutral proteases, and alkaline proteases in accordance with the classification based on the optimum pH. As the protease, one of these proteases may be used, or two or more of them may be used in combination. Among these proteases, acidic proteases and neutral proteases are preferable. In a case where the plant protein is an oat protein, among these proteases, acidic proteases (such as proteases derived from Aspergillus niger) and neutral proteases (such as proteases derived from Aspergillus oryzae) are preferable as the protease, neutral proteases are more preferable, and neutral proteases derived from Aspergillus oryzae are still more preferable, from the viewpoint of further improving the solubilization effect. Furthermore, in a case where the plant protein is an oat protein, among these proteases, neutral proteases (such as proteases derived from Aspergillus oryzae) are preferable as the protease, and neutral proteases derived from Aspergillus oryzae are more preferable, from the viewpoint of obtaining an effect of suppressing a change in taste. In cases where the plant protein is a pea protein, a chickpea protein, a rice protein, and/or an almond protein, among the proteases, neutral proteases and acidic proteases are preferable, and neutral proteases are preferable in some cases (such as a case where the plant protein is a rice protein, an almond protein, or a chickpea protein), from the viewpoint of further enhancing the effect of improving the solubility and/or the effect of suppressing a change in taste.

Examples of the protease include serine proteases, metal proteases, thiol proteases, and aspartic proteases in accordance with the classification based on catalytic mechanism. As the protease, one of these proteases may be used, or two or more of them may be used in combination.

The protease can be prepared with a known method. For example, the protease can be easily prepared with a method in which a microorganism as an origin of the protease is cultured and the produced protease is separated using a known means, or a method in which a gene recombination technique is used. As the protease, a commercially available product may be used. Examples of the commercially available protease include protease M "Amano" (an acidic protease derived from Aspergillus oryzae), protease HF "Amano" 150SD (an acidic protease derived from Aspergillus oryzae), protease A "Amano" (a neutral protease derived from Aspergillus oryzae), protease A "Amano" 2SD (a neutral protease derived from Aspergillus oryzae), acidic protease UF "Amano" SD (an acidic protease derived from Aspergillus niger), protease N "Amano" G (a neutral protease derived from Bacillus subtilis), PROTIN SD-NY10 (a neutral protease derived from Bacillus amyloliquefaciens), and THERMOASE PC10F (a neutral protease derived from Bacillus stearothermophilus) manufactured by Amano Enzyme Inc. and SUMIZYME MP (an alkaline protease derived from Aspergillus melleus) and SUMIZYME FP-G (an alkaline protease derived from Aspergillus oryzae) manufactured by SHIN NIHON CHEMICAL CO., LTD.

The amount of the protease used is not particularly limited, and is, for example, 0.0005 U or more, or 0.001 U or more with respect to 1 g of the plant protein. The amount of the protease used with respect to 1 g of the plant protein is preferably 0.003 U or more, 0.005 U or more, 0.01 U or more, 0.03 U or more, 0.05 U or more, 0.1 U or more, 0.2 U or more, 0.5 U or more, 1 U or more, 2 U or more, 3 U or more, 4 U or more, 6 U or more, 8 U or more, or 10 U or more from the viewpoint of further improving the solubilization effect or further improving the solubilization effect and the property of suppressing a change in taste. The upper limit of the range of the amount of the protease used with respect to 1 g of the plant protein is not particularly limited, and is, for example, 100 U or less, 90 U or less, 80 U or less, 70 U or less, 65 U or less, 60 U or less, 50 U or less, 40 U or less, 30 U or less, 20 U or less, 15 U or less, 10 U or less, 8 U or less, 7 U or less, or 6 U or less.

In a case where the plant protein is an oat protein, the preferred amount of the protease used with respect to 1 g of the oat protein is, for example, 0.01 U or more or 0.03 U or more, preferably 0.05 U or more, 0.1 U or more, or 0.2 U or more, more preferably 0.5 U or more, 1 U or more, or 2 U or more, and still more preferably 4 U or more, 6 U or more, 8 U or more, or 10 U or more from the viewpoint of further improving the solubilization effect and/or further improving the property of suppressing a change in taste. The preferred upper limit of the range of the amount of the protease used with respect to 1 g of the oat protein is, for example, 100 U or less, 90 U or less, 80 U or less, or 70 U or less, preferably 65 U or less, more preferably 40 U or less, still more preferably 30 U or less, even more preferably 20 U or less, and still even more preferably 15 U or less, 10 U or less, or 7 U or less from the viewpoint of further improving the solubilization effect.

In a case where the plant protein is a pea protein, a chickpea protein, a rice protein, and/or an almond protein, the preferred amount of the protease used with respect to 1 g of these plant proteins is, for example, 0.0005 U or more or 0.003 U or more, preferably 0.005 U or more, 0.01 U or more, or 0.03 U or more, more preferably 0.05 U or more, still more preferably 0.1 U or more, and still even more preferably 0.5 U or more, 1 U or more, 3 U or more, or 4 U or more from the viewpoint of further improving the solubilization effect and/or further improving the property of suppressing a change in taste. The preferred upper limit of the range of the amount of the protease used with respect to 1 g of these plant proteins is, for example, 50 U or less or 30 U or less, preferably 20 U or less or 10 U or less, more preferably 8 U or less, and still more preferably 6 U or less from the viewpoint of further improving the solubilization effect and/or further improving the property of suppressing a change in taste.

The amount of the protease used with respect to 1 g of the plant protein ingredient is, for example, 0.0001 U or more, or 0.00013 U or more. The amount of the protease used with respect to 1 g of the plant protein ingredient is preferably 0.0003 U or more, 0.0007 U or more, 0.0013 U or more, 0.003 U or more, 0.004 U or more, or 0.007 U or more, and more preferably 0.008 U or more, 0.01 U or more, 0.025 U or more, 0.05 U or more, 0.07 U or more, 0.1 U or more, 0.25 U or more, 0.5 U or more, 0.75 U or more, 0.8 U or more, 1 U or more, 1.3 U or more, 2 U or more, or 3 U or more from the viewpoint of further improving the solubilization effect or further improving the solubilization effect and the property of suppressing a change in taste. The upper limit of the range of the amount of the protease used with respect to 1 g of the plant protein ingredient is not particularly limited, and is, for example, 20 U or less, 15 U or less, 11 U or less, 10 U or less, 8.7 U or less, 8 U or less, 5 U or less, 4 U or less, 3 U or less, 2.5 U or less, 2 U or less, 1.5 U or less, 1.3 U or less, 0.9 U or less, 0.5 U or less, 0.3 U or less, 0.1 U or less, 0.05 U or less, 0.03 U or less, 0.01 U or less, or 0.005 U or less.

In a case where the plant protein is an oat protein, the preferred amount of the protease used with respect to 1 g of the oat (in terms of the amount of whole oat grains) is, for example, 0.002 U or more or 0.004 U or more, preferably 0.007 U or more, 0.01 U or more, or 0.025 U or more, more preferably 0.07 U or more, 0.1 U or more, or 0.25 U or more, and still more preferably 0.5 U or more, 0.75 U or more, 1 U or more, or 1.3 U or more from the viewpoint of further improving the solubilization effect or further improving the solubilization effect and the property of suppressing a change in taste. The preferred upper limit of the range of the amount of the protease used with respect to 1 g of the oat (in terms of the amount of whole oat grains) is, for example, 20 U or less, 15 U or less, or 10 U or less, preferably 8.7 U or less, more preferably 5 U or less, still more preferably 4 U or less, even more preferably 2.5 U or less, and still even more preferably 2 U or less, 1.3 U or less, or 0.9 U or less from the viewpoint of further improving the solubilization effect.

In a case where the plant protein is a rice protein, the preferred amount of the protease used with respect to 1 g of the rice (in terms of the amount of dry unpolished rice powder) is, for example, 0.0001 U or more, preferably 0.0003 U or more, and more preferably 0.003 U or more from the viewpoint of further enhancing the effect of improving the solubility and/or the effect of suppressing a change in taste. The preferred upper limit of the range of the amount of the protease used with respect to 1 g of the rice (in terms of the amount of dry unpolished rice powder) is, for example, 2 U or less, 0.5 U or less, 0.1 U or less, 0.05 U or less, 0.01 U or less, or 0.005 U or less.

In a case where the plant protein is an almond protein, the preferred amount of the protease used with respect to 1 g of the almond (in terms of the amount of almond powder) is, for example, 0.001 U or more or 0.003 U or more, and preferably 0.006 U or more or 0.008 U or more from the viewpoint of further enhancing the effect of improving the solubility and/or the effect of suppressing a change in taste. The preferred upper limit of the range of the amount of the protease used with respect to 1 g of the almond (in terms of the amount of almond powder) is, for example, 2 U or less, 0.5 U or less, 0.1 U or less, 0.05 U or less, or 0.03 U or less.

In a case where the plant protein is a chickpea protein, the preferred amount of the protease used with respect to 1 g of the chickpea is preferably 0.0003 U or more, more preferably 0.003 U or more, and still more preferably 0.008 U or more, 0.05 U or more, 0.1 U or more, 0.5 U or more, or 0.8 U or more from the viewpoint of further enhancing the effect of improving the solubility and/or the effect of suppressing a change in taste. The preferred upper limit of the range of the amount of the protease used with respect to 1 g of the chickpea is, for example, 10 U or less, 5 U or less, 3 U or less, 2 U or less, 0.5 U or less, 0.1 U or less, 0.05 U or less, or 0.03 U or less.

In a case where the plant protein is a pea protein, the preferred amount of the protease used with respect to 1 g of the pea is, for example, 0.008 U or more, more preferably 0.05 U or more, and still more preferably 0.1 U or more, 0.5 U or more, 1 U or more, 2 U or more, or 3 U or more from the viewpoint of further enhancing the effect of improving the solubility and/or the effect of suppressing a change in taste. The preferred upper limit of the range of the amount of the protease used with respect to 1 g of the pea is, for example, 50 U or less, and from the viewpoint of further enhancing the effect of improving the solubility and/or the effect of suppressing a change in taste, the upper limit is preferably 20 U or less, 15 U or less, 12 U or less, or 10 U or less, more preferably 8 U or less, still more preferably 5 U or less, and still even more preferably 4 U or less, 2 U or less, 1.5 U or less, 0.5 U or less, or 0.3 U or less.

The ratio of the amount of the protease used to the amount of the protein deamidase used is determined by the above-described amount of each enzyme used, but from the viewpoint of further improving the solubilization effect or further improving the solubilization effect and the property of suppressing a change in taste, the amount of the protease used with respect to 1 U of the protein deamidase is, for example, 0.0001 U or more, 0.0005 U or more, or 0.001 U or more, more preferably 0.002 U or more, 0.003 U or more, 0.006 U or more, 0.015 U or more, 0.016 U or more, 0.03 U or more, 0.05 U or more, 0.067 U or more, 0.1 U or more, 0.15 U or more, 0.16 U or more, 0.3 U or more, 0.5 U or more, 0.6 U or more, 1 U or more, 1.3 U or more, 1.5 U or more, 1.8 U or more, 2 U or more, 2.6 U or more, or 3.3 U or more. The upper limit of the range of the amount of the protease used with respect to 1 U of the protein deamidase is, for example, 50 U or less, 40 U or less, 33 U or less, 30 U or less, 26 U or less, 25 U or less, 20 U or less, 15 U or less, 13 U or less, 10 U or less, 8 U or less, 7 U or less, 5 U or less, 3.5 U or less, 3 U or less, or 2.3 U or less.

In a case where the plant protein is an oat protein, the preferred amount of the protease used with respect to 1 U of the protein deamidase is, for example, 0.001 U or more, 0.005 U or more, 0.01 U or more, or 0.016 U or more, preferably 0.02 U or more, 0.03 U or more, or 0.067 U or more, more preferably 0.16 U or more, 0.3 U or more, or 0.6 U or more, and still more preferably 1.3 U or more, 2 U or more, 2.6 U or more, or 3.3 U or more from the viewpoint of further improving the solubilization effect or further improving the solubilization effect and the property of suppressing a change in taste. The preferred upper limit of the range of the amount of the protease used with respect to 1 U of the protein deamidase is, for example, 50 U or less, 40 U or less, 30 U or less, or 25 U or less, preferably 20 U or less, more preferably 13 U or less, still more preferably 10 U or less, even more preferably 7 U or less, and still even more preferably 5 U or less, 3 U or less, or 2.3 U or less from the viewpoint of further improving the solubilization effect.

In a case where the plant protein is a pea protein, a chickpea protein, a rice protein, and/or an almond protein, the preferred amount of the protease used with respect to 1 U of the protein deamidase is, for example, 0.0001 U or more, 0.0005 U or more, or 0.001 U or more, more preferably 0.0015 U or more or 0.002 U or more, still more preferably 0.006 U or more, and still even more preferably 0.015 U or more, 0.05 U or more, 0.1 U or more, 0.15 U or more, 0.5 U or more, 1 U or more, 1.5 U or more, or 1.8 U or more from the viewpoint of further improving the solubilization effect and/or further improving the property of suppressing a change in taste. The preferred upper limit of the range of the amount of the protease used with respect to 1 U of the protein deamidase is, for example, 20 U or less, and from the viewpoint of further enhancing the effect of improving the solubility and/or the effect of suppressing a change in taste, the upper limit is preferably 15 U or less, more preferably 10 U or less, still more preferably 8 U or less or 7 U or less, and still even more preferably 5 U or less or 3.5 U or less.

The activity of the protease is measured with the Folin method using casein as a substrate. That is, the activity of the protease is defined so that when an enzyme reaction is carried out with a conventional method using casein as a substrate, the amount of the enzyme that increases a folin reagent-coloring substance by an amount equivalent to 1 $\mu$g of tyrosine per minute is 1 unit (1 U).

1-4. Reaction Conditions, etc.

In the step of treating a plant protein-containing liquid composition with a protease and a protein deamidase, the order of actions of the protease and the protein deamidase is not particularly limited, and the enzymes may be sequentially made to act in any order, or both the enzymes may be simultaneously made to act. However, from the viewpoint of further improving the solubilization effect, it is particularly preferable that the plant protein-containing liquid composition be preferably treated with the protease and then treated with the protein deamidase.

The treatment temperature with the protease and the protein deamidase is not particularly limited, and can be appropriately determined by those skilled in the art according to, for example, the optimum temperature of each enzyme to be used and/or the thermal property of the plant protein-containing liquid composition. For example, the treatment temperature is 40 to 70°C, and preferably 48 to 62°C. Specifically, the treatment temperature with the protein deamidase is, for example, 40 to 60°C, preferably 45 to 55°C, and more preferably 48 to 52°C. The treatment temperature with the protease is, for example, 40 to 70°C, preferably 50 to 65°C, and more preferably 58 to 62°C.

The reaction time of the enzyme treatment of the plant protein-containing liquid composition is not particularly limited, and is to be appropriately determined according to the scale of the amount of the composition to be treated, the timing of adding the enzymes, and the like. For example, the reaction time is 30 minutes or more, and preferably 50 minutes or more. The upper limit of the range of the reaction time of the enzyme treatment is not particularly limited, and is, for example, 12 hours or less, 6 hours or less, 3 hours or less, 2.5 hours or less, or 2 hours or less. Specifically, the treatment time with the protease is, for example, 5 minutes to 2 hours, preferably 5 minutes to 1 hour, and more preferably 35 to 55 minutes or 40 minutes to 1.5 hours. The treatment time with the protein deamidase is preferably 20 minutes to 6 hours, and more preferably 40 minutes to 1.5 hours.

The plant protein-containing liquid composition after completion of the enzyme treatment is subjected to an enzyme deactivation step as necessary, cooled, and further subjected to a post-treatment step such as filtration as necessary to obtain a processed plant protein-containing liquid composition.

The obtained processed plant protein-containing liquid composition can be further subjected to a drying step and thus prepared as a solid plant protein composition in which the solubility in water is improved or a change in taste is suppressed while the solubility in water is improved. The method of drying is not particularly limited, and examples of the method include freeze-drying, vacuum drying, and spray drying. The solid plant protein composition has a form of, for example, a powder, fine particles, or granules.

2. Solubilizer for Plant Protein-Containing Liquid Composition

Combination of a protein deamidase and a protease can improve the solubility of a plant protein-containing liquid composition. Therefore, the present invention also provides a solubilizer, for a plant protein-containing liquid composition, including a protease and a protein deamidase.

In the above-described solubilizer, the kind and the amount of the component used and the like are as shown in the above-described item "1. Method for Producing Processed Plant Protein-Containing Liquid Composition".

3. Solubilizer for Plant Protein-Containing Liquid Composition to Be Treated with Protein Deamidase

A neutral protease or a protease derived from a filamentous fungus can solubilize a plant protein-containing liquid composition to be treated with a protein deamidase while a change in taste of the plant protein-containing liquid composition is suppressed. Specifically, when a plant protein-containing liquid composition to be treated with a protein deamidase is solubilized using a neutral protease or a protease derived from a filamentous fungus, the composition can be solubilized without a change in taste that may be usually caused by protease treatment. Therefore, the present invention also provides a solubilizer that includes a neutral protease or a protease derived from a filamentous fungus and is to be used for solubilizing a plant protein-containing liquid composition to be treated with a protein deamidase while a change in taste of the plant protein-containing liquid composition is suppressed.

Preferred examples of the solubilizer include solubilizers that contain a neutral protease and are used for solubilizing an oat protein-containing liquid composition to be treated with a protein deamidase while a change in taste of the composition is suppressed, and solubilizers that contain a protease derived from Aspergillus oryzae and are used for solubilizing an oat protein-containing liquid composition to be treated with a protein deamidase while a change in taste of the composition is suppressed.

Other preferred examples of the solubilizer include solubilizers that contain a protease derived from a filamentous fungus and are used for solubilizing a plant protein-containing liquid composition to be treated with a protein deamidase while a change in taste of the composition is suppressed, and the plant protein is a pea protein, a chickpea protein, a rice protein, and/or an almond protein.

In the solubilizer, the term "solubilizing" means giving the plant protein-containing liquid composition a property of having a larger amount of protein dissolved in water than in a case where the composition is solubilized only with the protein deamidase. Specific aspects of use of the solubilizer include all of aspects in which the solubilizer and the protein deamidase are simultaneously used to treat the plant protein-containing liquid composition, aspects in which the plant protein-containing liquid composition is treated with the protein deamidase and then treated with the solubilizer, and aspects in which the plant protein-containing liquid composition is treated with the solubilizer and then treated with the protein deamidase.

In the above-described solubilizer, the kind and the amount of the component used and the like are as shown in the above-described item "1. Method for Producing Processed Plant Protein-Containing Liquid Composition".

EXAMPLES

Hereinafter, the present invention will be specifically described with reference to Examples, but the present invention is not to be construed as being limited to the following Examples.

[Used Enzyme]

. KSSD-8 (Kleistase SD8): $\alpha$-Amylase derived from Bacillus amyloliquefaciens
· PR-ASD (Protease A "Amano" SD): Neutral protease derived from Aspergillus oryzae
· TH-PC10F (THERMOASE PC10F): Metal protease derived from Geobacillus stearothermophilus
· PR-UFSD (Acid Protease UF "Amano" SD): Acidic protease derived from Aspergillus niger
· PR-HF150SD (Protease HF "Amano" 150SD): Acidic protease derived from Aspergillus oryzae
· PG-500 (Protein-glutaminase"Amano"500): Protein-glutaminase (protein deamidase) derived from Chryseobacterium proteolyticum

[Method of Measuring Enzyme Activity]

(1) Method of Measuring Protease Activity

After heating 5 mL of a 0.6% (v/w) casein solution (0.05 mol/L sodium hydrogen phosphate, pH 8.0 [in the case of TH-PC10F] or pH 6.0 [in the case of PR-ASD]) or a 0.6% (v/w) casein solution (0.7% (v/w) lactic acid, pH 3.0 [in the case of PR-UFSD or PR-HF150SD]) at 37°C for 10 minutes, 1 mL of a sample solution containing a protease was added, and the mixture was immediately shaken. After this liquid was allowed to stand at 37°C for 10 minutes, 5 mL of a trichloroacetic acid reagent (trichloroacetic acid containing 1.8% trichloroacetic acid, 1.8% sodium acetate and 0.33 mol/L acetic acid [in the case of TH-PC10F], or 0.44 mol/L trichloroacetic acid [in the case of PR-ASD, PR-UFSD, or PR-HF150SD]) was added, and the mixture was shaken, allowed to stand at 37°C for 30 minutes again, and filtered. The first filtrate (3 mL) was removed, and the next filtrate (2 mL) was measured out, 5 mL of a 0.55 mol/L sodium carbonate reagent and 1 mL of a folin reagent (1 → 3) were added, and the mixture was well shaken and allowed to stand at 37°C for 30 minutes. This liquid (enzyme reaction liquid) was measured using water as a control to determine the absorbance AT at a wavelength of 660 nm.

Separately, a liquid (blank) was obtained by an operation similar to that performed to obtain the above-described enzyme reaction liquid, except the following procedure. A 1 mL of a sample solution containing a protease was measured out, 5 mL of a trichloroacetic acid reagent (trichloroacetic acid containing 1.8% trichloroacetic acid, 1.8% sodium acetate and 0.33 mol/L acetic acid [in the case of TH-PC10F], or 0.44 mol/L trichloroacetic acid [in the case of PR-ASD, PR-UFSD, or PR-HF150SD]) was added, the mixture was shaken, then 5 mL of a casein solution having a measured pH set for each sample was added, and the mixture was immediately shaken and allowed to stand at 37°C for 30 minutes. The obtained liquid (blank) was measured to determine the absorbance AB.

The amount of the enzyme that increased a folin reagent-coloring substance by an amount equivalent to 1 μg of tyrosine per minute was defined as 1 unit (1 U).

A 1 mg/mL tyrosine standard stock solution (0.2 mol/L hydrochloric acid) was measured out in amounts of 1 mL, 2 mL, 3 mL, and 4 mL, and to each measured out solutions, a 0.2 mol/L hydrochloric acid reagent was added so that the resulting liquid had an amount of 100 mL. Each liquid was measured out in an amount of 2 mL, 5 mL of a 0.55 mol/L sodium carbonate reagent and 1 mL of a folin reagent (1 → 3) were added, and the mixture was immediately shaken and allowed to stand at 37°C for 30 minutes. These liquids were each measured using, as a control, a liquid obtained by measuring out 2 mL of a 0.2 mol/L hydrochloric acid reagent and performing an operation similar to the above-described operation to determine the absorbances A1, A2, A3, and A4 at a wavelength of 660 nm. A calibration curve was prepared by plotting the absorbances A1, A2, A3, and A4 on the vertical axis and plotting the amount of tyrosine (μg) in 2 mL of each liquid on the horizontal axis, and thus the amount of tyrosine (μg) with respect to the absorbance difference 1 was determined.

[Math. 1]

$$\text{Protease activity (U/g, U/mL)} = (AT - AB) \times F \times 11/2 \times 1/10 \times 1/M$$

AT: Absorbance of enzyme reaction liquid
AB: Absorbance of blank
F: Amount (μg) of tyrosine when absorbance difference is 1, determined from tyrosine calibration curve
11/2: Conversion factor to total liquid amount after stop of reaction
1/10: Conversion factor to value per minute of reaction time
M: Amount (g or mL) of sample in 1 mL of sample solution

(2) Method of Measuring Protein Deamidase Activity

To 1 mL of a 0.2 M phosphate buffer (pH 6.5) containing 30 mM Z-Gln-Gly, 0.1 mL of a sample solution containing a protein deamidase was added, the mixture was allowed to stand at 37°C for 10 minutes, and then 1 mL of a 0.4 M TCA solution was added to stop the reaction. To 1 mL of a 0.2 M phosphate buffer (pH 6.5) containing 30 mM Z-Gln-Gly, 1 mL of a 0.4 M TCA reagent was added, 0.1 mL of a sample solution containing a protein deamidase was further added, and the mixture was allowed to stand at 37°C for 10 minutes as a blank.

The solution obtained above was measured using Ammonia Test Wako (FUJIFILM Wako Pure Chemical Corporation) to determine the amount of ammonia generated in the reaction liquid. A calibration curve representing the relationship between the ammonia concentration and the absorbance (630 nm) was prepared using an ammonia standard solution (ammonium chloride), and from the calibration curve, the ammonia concentration in the reaction liquid was determined.

The amount of the enzyme that produces 1 μmol of ammonia per minute was defined as 1 unit (1 U), and the activity of the protein deamidase was calculated from the following formula. In the formula, the reaction liquid amount is 2.1, the enzyme solution amount is 0.1, and Df is a dilution rate of the enzyme solution. Furthermore, 17.03 is a molecular weight of ammonia.

[Math. 2]

$$\text{Protein deamidase activity (U/mL)} = \text{ammonia concentration in reaction liquid (mg/L)}$$

$$\times \ (1/17.03) \times (\text{reaction liquid amount/enzyme solution amount}) \times (1/10) \times Df$$

(3) Method of Measuring α-Amylase

After heating 10 mL of a 1% potato starch substrate solution (0.1 mol/L acetic acid (pH 5.0)) at 37°C for 10 minutes, 1 mL of a sample solution containing α-amylase was added, and the mixture was immediately shaken. After this solution was allowed to stand at 37°C for 10 minutes, 1 mL of this solution was added to 10 mL of a 0.1 mol/L hydrochloric acid reagent, and the mixture was immediately shaken. Next, 0.5 mL of the resulting liquid was measured out, 10 mL of a 0.0002 mol/L iodine reagent (Japanese Pharmacopoeia) was added, and the mixture was shaken and then measured using water as a control to determine the absorbance (AT) at a wavelength of 660 nm. Separately, a similar operation was performed except that 1 mL of water was added instead of the sample solution, and the absorbance (AB) was measured. The amount of the enzyme that reduces coloring of potato starch due to iodine by 10% per minute was defined as 1 unit (1 U).

[Math. 3]

$$\alpha\text{-Amylase activity (U/g, U/mL)} = (AB - AT)/AB \times 1/W$$

AT: Absorbance of reaction liquid
AB: Absorbance of blank liquid
W: Amount (g or mL) of sample in 1 mL of sample solution

[Test Example 1]

(1) Method

To 90 mL of water, 10 g of oat flour (equivalent to 10 g of an oat ingredient (whole grains) and having a protein content of 1.4 g) and 50 mg (40 U/1 g of oat flour) of $\alpha$-amylase KSSD-8 were added and suspended, protease PR-ASD was added in an amount shown in Table 1, the mixture was stirred for 5 minutes and then treated at 60°C for 45 minutes, then protein-glutaminase PG-500 was added in an amount shown in Table 1, and the resulting mixture was treated at 50°C for 1 hour, boiled for 10 minutes, and cooled to room temperature. Thus, a processed oat milk was obtained.

(2) Evaluation of Solubilization

The obtained processed oat milk was centrifuged at 15000 rpm for 15 minutes, then the supernatant was collected 2 times so as not to take the cloudy upper layer, and the supernatant was measured with the Bradford method to determine the protein concentration (mg/mL). The protein concentration obtained in Comparative Example 1 in which a protein deamidase was used singly was regarded as 1, and thus the relative concentration of the protein concentration obtained in each Example was calculated. Table 1 shows the results. A processed oat milk obtained by a similar treatment using none of a protease and a protein deamidase had a protein concentration determined as described above of almost 0 mg/L, but in Comparative Example 1, the protein concentration was improved to more than about 2 mg/mL.

(3) Evaluation of Property of Suppressing Change in Taste

From the viewpoint of creamy feeling and milk feeling, the tastes of the processed oat milks in Examples were compared using, as a reference, the taste (perceived as creamy feeling and milk feeling) of the processed oat milk in Comparative Example 1 in which a protein deamidase was used singly, and all of the scores corresponding to the following five items were added for each processed oat milk to obtain an index of the property of suppressing a change in taste. The highest score of this index is +1, and the lower the score indicates lower property of suppressing a change in taste. The term "creamy feeling" refers to a taste that is perceived when a processed oat milk is put into the mouth and perceived to be rich due to feeling such that the processed oat milk remains on the tongue by combination of the fineness and the viscosity of the processed oat milk. The term "milk feeling" refers to a milky flavor. Table 1 shows the results. Regarding the taste of the processed oat milk obtained by a similar treatment using none of a protease and a protein deamidase, no creamy feeling was perceived with the rough texture.

| | |
|---|---|
| No change occurred in taste | +1 point |
| Creaminess was slightly reduced and slight lightness was generated | -1 point |
| Creaminess was reduced and lightness was generated | -2 points |
| Milk feeling was slightly reduced | -1 point |
| Milk feeling was reduced | -2 points |

[Table 1]

| | PR-ASD | | PG-500 | | Protein concentration | Property of suppressing change in taste (point) |
|---|---|---|---|---|---|---|
| | (U/1 g of oat) | (U/1 g of protein) | (U/1 g of oat) | (U/1 g of protein) | (relative concentration) | |
| Comparative Example 1 | 0 | 0 | 0.4 | 3 | 1 | (Reference) |
| Example 1 | 0.0084 | 0.064 | 0.4 | 3 | 1.28 | +1 |
| Example 2 | 0.084 | 0.64 | 0.4 | 3 | 1.40 | +1 |
| Example 3 | 0.84 | 6.4 | 0.4 | 3 | 1.48 | +1 |
| Example 4 | 8.4 | 64 | 0.4 | 3 | 1.20 | +1 |

As is apparent from Table 1, treating an oat milk with a protease and a protein deamidase further improved the solubility. In the case of using PR-ASD, the taste was not changed while the solubility was improved, and thus an excellent property of suppressing a change in taste was also recognized.

[Test Example 2]

A processed oat milk was prepared in the same manner as in Test Example 1 except that the enzyme shown in Table 2 was used as the protease in an amount indicated, and the solubilization and the property of suppressing a change in taste were evaluated. Table 2 shows the results.

[Table 2]

| | TH-PC10F | | PG-500 | | Protein concentration | Property of suppressing |
|---|---|---|---|---|---|---|
| | (U/1 g of oat) | (U/1 g of protein) | (U/1 g of oat) | (U/1 g of protein) | (relative concentration) | change in taste (point) |
| Comparative Example 1 | 0 | 0 | 0.4 | 3 | 1 | (Reference) |
| Example 5 | 0.014 | 0.11 | 0.4 | 3 | 1.47 | -3 |
| Example 6 | 0.14 | 1.1 | 0.4 | 3 | 1.35 | -3 |
| Example 7 | 1.4 | 11 | 0.4 | 3 | 1.59 | -4 |

As is apparent from Table 2, treating an oat milk with a protease and a protein deamidase further improved the solubility.

[Test Example 3]

A processed oat milk was prepared in the same manner as in Test Example 1 except that the enzyme shown in Table 3 was used as the protease in an amount indicated, and the solubilization and the property of suppressing a change in taste were evaluated. Table 3 shows the results.

[Table 3]

| | PR-UFSD | | PG-500 | | Protein concentration | Property of suppressing |
|---|---|---|---|---|---|---|
| | (U/1 g of oat) | (U/1 g of protein) | (U/1 g of oat) | (U/1 g of protein) | (relative concentration) | change in taste (point) |
| Comparative Example 1 | 0 | 0 | 0.4 | 3 | 1 | (Reference) |
| Example 8 | 0.028 | 0.21 | 0.4 | 3 | 1.13 | -3 |
| Example 9 | 0.28 | 2.1 | 0.4 | 3 | 1.07 | -3 |

As is apparent from Table 3, treating an oat milk with a protease and a protein deamidase further improved the solubility.

[Test Example 4]

(1) Production of Processed Plant Milk

(1-1) Production of Processed Rice Milk

In 50 g of water, 15 g of an unpolished rice powder (protein content: 7.1 wt%) was dispersed, 50 mg of an amylase and a protease of the type and the amount shown in Table 4 were added, the mixture was treated at 60°C for 1 hour, then a protein deamidase of the amount shown in Table 4 was added, and the resulting mixture was treated at 50°C for 1 hour. The treated rice milk composition was boiled for 10 minutes, allowed to dissipate heat on ice, and cooled to obtain a processed rice milk.

(1-2) Production of Processed Almond Milk

In 60 g of water, 10 g of an almond powder (protein content: 19.6 wt%) was dispersed to prepare an almond milk, a protease of the type and the amount shown in Table 4 was added, the mixture was treated at 60°C for 80 minutes, then a protein deamidase of the amount shown in Table 4 was added, and the resulting mixture was treated at 50°C for 1 hour. The treated almond milk composition was boiled for 10 minutes, allowed to dissipate heat on ice, and cooled to obtain a processed almond milk.

(1-3) Production of Processed Chickpea Milk

In water, 300 g of a chickpea (protein content: 20 wt%) was immersed overnight, and the resulting product was ground with a mixer. The total amount was adjusted to 2,700 mL with water to obtain a chickpea milk. The chickpea milk was divided into portions of 100 mL, a protease of the type and the amount shown in Table 5 was added, the mixture was treated at 60°C for 1 hour, then a protein deamidase of the amount shown in Table 5 was added, and the resulting mixture was treated at 50°C for 1 hour. The treated chickpea milk composition was boiled for 10 minutes, allowed to dissipate heat on ice, and cooled to obtain a processed chickpea milk.

(1-4) Production of Processed Pea Milk

To 10 g of a pea protein ingredient (protein content: 79 wt%), 3.6 g of a sunflower oil was added, water was further added to adjust the total amount to 240 mL, and then the mixture was homogenized at 14,000 rpm for 3 minutes to prepare a pea milk. To the pea milk, a protease and a protein deamidase of the types and the amounts shown in Table 6 were added, and the resulting mixture was treated at 50°C for 2 hours. The treated pea milk composition was boiled for 15 minutes, allowed to dissipate heat on ice, and cooled to obtain a processed pea milk.

(2) Evaluation of Effect of Improving Solubility

The obtained processed plant milk was centrifuged at 15000 rpm for 15 minutes, then the supernatant was collected 2 times so as not to take the cloudy upper layer, and the supernatant was measured with the Bradford method to determine the protein (soluble in water) concentration (mg/mL). The protein concentrations obtained in Comparative Examples 2, 3, 4, and 5 in which a protein deamidase was used singly were each regarded as 1, and thus the relative concentration of the protein concentration obtained in each Example was calculated. Tables 4 to 6 show the results. The relative protein concentration was classified in accordance with the following criteria, and the degree of the effect of improving the solubility was evaluated. Tables 4 to 6 show the results.

++++ Relative protein concentration is 1.15 or more
+++ Relative protein concentration is 1.1 or more and less than 1.15
++ Relative protein concentration is 1.05 or more and less than 1.1
+ Relative protein concentration is 1 or more and less than 1.05
- Relative protein concentration is less than 1

(3) Evaluation of Effect of Suppressing Change in Taste

The tastes of the processed plant milks in Examples were compared using, as a reference, the tastes of the processed plant milks in Comparative Examples 2, 3, 4, and 5 in which a protein deamidase was used singly, and a case where no change occurred in the taste was evaluated as "○", and a case where a change occurred in the taste was evaluated as "×", Tables 4 to 6 show the results.

[Table 4]

| | | | Comparative Example 2 | Example 10 | Example 11 | Comparative Example 3 | Example 12 |
|---|---|---|---|---|---|---|---|
| Food ingredient | Rice | Contained protein (*1) | 1.64% | 1.64% | 1.64% | - | - |
| | Almond | | - | - | - | 2.80% | 2.80% |
| | Chickpea | | - | - | - | - | - |
| | Pea | | - | - | - | - | - |
| Protease derived from filamentous fungus | PR-ASD | (*1) | - | 0.00164 ppm | 0.0164 ppm | - | 0.028 ppm |
| | | (*2) | - | 0.005 U | 0.05 U | - | 0.05 U |
| | | (*3) | - | 0.00036 U | 0.0036 U | - | 0.0098 U |
| Protein deamidase | PG-500 | (*1) | 82 ppm | 82 ppm | 82 ppm | 140 ppm | 140 ppm |
| | | (*2) | 2.5 U | 2.5 U | 2.5 U | 2.5 U | 2.5 U |
| | | (*3) | 0.18 U | 0.18 U | 0.18 U | 0.49 U | 0.49 U |
| Effect of improving solubility | Relative protein concentration | | 1 | 1.16 | 1.19 | 1 | 1.16 |
| | Evaluation | | Reference | ++++ | ++++ | Reference | ++++ |
| Effect of suppressing change in taste | Evaluation | | Reference | ○ | ○ | Reference | ○ |

(*1) Weight concentration (in plant milk composition)  (*2) Activity value with respect to 1 g of protein
(*3) Activity value with respect to 1 g of food ingredient

[Table 5]

| | | | Comparative Example 4 | Example 13 | Example 14 | Example 15 | Example 16 |
|---|---|---|---|---|---|---|---|
| Food ingredient | Rice | Contained protein (*1) | - | - | - | - | - |
| | Almond | | - | - | - | - | - |
| | Chickpea | | 2.22% | 2.22% | 2.22% | 2.22% | 2.22% |
| | Pea | | - | - | - | - | - |
| Protease derived from filamentous fungus | PR-ASD | (*1) | - | 0.00222 ppm | 0.0222 ppm | 2.22 ppm | - |
| | | (*2) | - | 0.005 U | 0.05 U | 5 U | - |
| | | (*3) | - | 0.001 U | 0.01 U | 1 U | - |
| | PR-HF 150SD | (*1) | - | - | - | - | 0.0222 ppm |
| | | (*2) | - | - | - | - | 0.15 U |
| | | (*3) | - | - | - | - | 0.03 U |
| Protein deamidase | PG-500 | (*1) | 111 ppm | 111 ppm | 111 ppm | 111 ppm | 111 ppm |
| | | (*2) | 2.5 U | 2.5 U | 2.5 U | 2.5 U | 2.5 U |
| | | (*3) | 0.5 U | 0.5 U | 0.5 U | 0.5 U | 0.5 U |
| Effect of improving solubility | Relative protein concentration | | 1 | 1.04 | 1.12 | 1.13 | 1.07 |
| | Evaluation | | Reference | + | +++ | +++ | ++ |
| Effect of suppressing change in taste | Evaluation | | Reference | ○ | ○ | ○ | ○ |

(*1) Weight concentration (in plant milk composition)   (*2) Activity value with respect to 1 g of protein
(*3) Activity value with respect to 1 g of food ingredient

[Table 6]

| | | | Comparative Example 5 | Example 17 | Example 18 | Example 19 | Example 20 | Example 21 | Example 22 |
|---|---|---|---|---|---|---|---|---|---|
| Food ingredient | Rice | Contained protein (*1) | - | - | - | - | - | - | - |
| | Almond | | - | - | - | - | - | - | - |
| | Chickpea | | - | - | - | - | - | - | - |
| | Pea | | 3.29% | 3.29% | 3.29% | 3.29% | 3.29% | 3.29% | 3.29% |
| Protease derived from filamentous fungus | PR-ASD | (*1) | - | 3.29 ppm | - | - | - | - | - |
| | | (*2) | - | 5 U | - | - | - | - | - |
| | | (*3) | - | 3.95 U | - | - | - | - | - |
| | PR-HF 150SD | (*1) | - | - | 0.0329 ppm | 0.329 ppm | 3.29 ppm | - | - |
| | | (*2) | - | - | 0.15 U | 1.5 U | 15 U | - | - |
| | | (*3) | - | - | 0.12 U | 1.19 U | 11.9 U | - | - |
| Protease derived from bacterium | TH-PC10F | (*1) | - | - | - | - | - | 0.0329 ppm | 0.329 ppm |
| | | (*2) | - | - | - | - | - | 0.09 U | 0.9 U |
| | | (*3) | - | - | - | - | - | 0.071 U | 0.71 U |
| Protein deamidase | PG-500 | (*1) | 164.5 ppm | 164.5 ppm | 164.5 ppm | 164.5 ppm | 164.5 ppm | 164.5 ppm | 164.5 ppm |
| | | (*2) | 2.5 U | 2.5 U | 2.5 U | 2.5 U | 2.5 U | 2.5 U | 2.5 U |
| | | (*3) | 1.98 U | 1.98 U | 1.98 U | 1.98 U | 1.98 U | 1.98 U | 1.98 U |
| Effect of improving solubility | Relative protein concentration | | 1 | 1.05 | 1.06 | 1.06 | 1.01 | 1.02 | 1.03 |
| | Evaluation | | Reference | ++ | ++ | ++ | + | + | + |
| Effect of suppressing change in taste | Evaluation | | Reference | ○ | ○ | ○ | ○ | × | × |

(*1) Weight concentration (in plant milk composition)   (*2) Activity value with respect to 1 g of protein
(*3) Activity value with respect to 1 g of food ingredient

As is apparent from the comparison between Comparative Example 2 and Examples 10 and 11, the comparison between Comparative Example 3 and Example 12, the comparison between Comparative Example 4 and Examples 13 to 16, and the comparison between Comparative Example 5 and Examples 17 to 22, treating a plant milk with a protease and a protein deamidase enhanced the solubility of the protein of the plant milk (Examples 10 to 22). Furthermore, as is apparent from the comparison between Examples 10 to 20 and Examples 21 to 22, treatment with a protease derived from a filamentous fungus and a protein deamidase enhanced the solubility of the protein without a change in taste of the plant milk (Examples 10 to 20).

**Claims**

1. A method for producing a processed plant protein-containing liquid composition, the method comprising a step of treating a plant protein-containing liquid composition with a protease and a protein deamidase.

2. The method according to claim 1, wherein the plant protein-containing liquid composition is treated with the protease and then treated with the protein deamidase.

3. The method according to claim 1 or 2, wherein the protease is a protease derived from a filamentous fungus.

4. The method according to any one of claims 1 to 3, wherein the protease is derived from Aspergillus oryzae.

5. The method according to any one of claims 1 to 4, wherein the plant protein contains a protein of a plant selected from the group consisting of oats, peas, chickpeas, rice, and almonds.

6. The method according to claims 1 to 5, wherein the plant protein-containing liquid composition is a plant milk.

7. A solubilizer for a plant protein-containing liquid composition, the solubilizer comprising a protease and a protein deamidase.

8. A solubilizer comprising a neutral protease, the solubilizer to be used for solubilizing a plant protein-containing liquid composition to be treated with a protein deamidase while a change in taste of the plant protein-containing liquid composition is suppressed.

9. A solubilizer comprising a protease derived from a filamentous fungus, the solubilizer to be used for solubilizing a plant protein-containing liquid composition to be treated with a protein deamidase while a change in taste of the plant protein-containing liquid composition is suppressed.

## INTERNATIONAL SEARCH REPORT

| International application No. |
|---|
| **PCT/JP2021/041607** |

### A. CLASSIFICATION OF SUBJECT MATTER

*A23J 3/14*(2006.01)i; *A23J 3/34*(2006.01)i; *A23L 25/00*(2016.01)i; *A23L 11/60*(2021.01)i; *A23L 2/66*(2006.01)i; *A23L 2/38*(2021.01)i; *A23L 7/10*(2016.01)i; *A23L 29/00*(2016.01)i
FI:    A23J3/14; A23J3/34; A23L11/60; A23L7/10 A; A23L29/00; A23L25/00; A23L2/00 J; A23L2/38 J

According to International Patent Classification (IPC) or to both national classification and IPC

### B. FIELDS SEARCHED

Minimum documentation searched (classification system followed by classification symbols)

A23J3/14; A23J3/34; A23L7/10; A23L25/00; A23L29/00; A23L2/66; A23L2/38; A23L11/60

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2020/171106 A1 (AMANO ENZYME INC.) 27 August 2020 (2020-08-27)<br>examples | 1-9 |
| Y | SUPPAVORASATIT, Inthawoot, LEE, Soo-Yeun, CADWALLADER, Keith R. Effect of enzymatic protein deamidation on protein solubility and flavor binding properties of soymi. Journal of Food Science. 2013, pp. C1-C7<br>abstract, fig. 1 | 1-9 |
| Y | WO 2015/122424 A1 (FUJI OIL CO., LTD.) 20 August 2015 (2015-08-20)<br>paragraphs [0024], [0025] | 1-9 |
| Y | JP 2020-145927 A (AJINOMOTO KK) 17 September 2020 (2020-09-17)<br>paragraphs [0023]-[0026] | 1-9 |
| Y | JP 2006-6345 A (SAWA SANGYO KK) 12 January 2006 (2006-01-12)<br>paragraph [0010], example 4 | 1-9 |
| Y | WO 2020/208734 A1 (MIZKAN HOLDINGS CO., LTD.) 15 October 2020 (2020-10-15)<br>paragraphs [0057], [0058], [0062] | 1-9 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 December 2021** | **11 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/041607**

| Patent document<br>cited in search report | | | Publication date<br>(day/month/year) | Patent family member(s) | Publication date<br>(day/month/year) |
|---|---|---|---|---|---|
| WO | 2020/171106 | A1 | 27 August 2020 | (Family: none) | |
| WO | 2015/122424 | A1 | 20 August 2015 | (Family: none) | |
| JP | 2020-145927 | A | 17 September 2020 | (Family: none) | |
| JP | 2006-6345 | A | 12 January 2006 | US 6582739 B1<br>column 8, example 4<br>TW 234434 B<br>KR 10-0669478 B1 | |
| WO | 2020/208734 | A1 | 15 October 2020 | (Family: none) | |

Form PCT/ISA/210 (patent family annex) (January 2015)

**EP 4 245 149 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020171106 A
- JP 2000050887 A
- JP 2001218590 A
- WO 2006075772 A1
- WO 2015133590 A